Europäisches Patentamt

(19) **European Patent Office**

**Office européen des brevets**

(11) Publication number: **0 134 662**

**A1**

(12) # EUROPEAN PATENT APPLICATION

(21) Application number: **84304688.9**

(22) Date of filing: **09.07.84**

(51) Int. Cl.⁴: **C 12 P 21/02**
**C 12 N 15/00, C 12 N 9/48**
**C 07 K 7/10**

(30) Priority: **07.07.83 US 511766**
**28.06.84 US 625680**

(43) Date of publication of application:
**20.03.85 Bulletin 85/12**

(84) Designated Contracting States:
**AT BE CH DE FR GB IT LI LU NL SE**

(71) Applicant: **GENEX CORPORATION**
**6110 Executive Boulevard**
**Rockville Maryland 20852(US)**

(72) Inventor: **Anderson, David M.**
**13501 Grenoble Drive**
**Rockville, MD 20853(US)**

(72) Inventor: **Young, Debra A.**
**3024 Wisconsin Avenue**
**Apartment B106 Washington, D.C. 20016(US)**

(72) Inventor: **Kalk, Elizabeth Spencer**
**17204 Whites Road**
**Poolesville, MD 20837(US)**

(74) Representative: **Holmes, Michael John et al,**
**Frank B. Dehn & Co. Imperial House 15-19 Kingsway**
**London WC2B 6UZ(GB)**

(54) **Production of bovine calf chymosin.**

(57) A replicable plasmidic expression vehicle for expression of a polypeptide which is capable of undergoing autocatalytic cleavage to produce chymosin is disclosed. The expression vehicle contains a regulatory region which directs expression, in a host microorganism of the *trpB* gene or the *glyA* gene, and a DNA sequence, operably linked to the regulatory region, comprising a sequence which encodes an N-terminal fragment of the β subunit of tryptophan synthetase or an N-terminal fragment of serine hydroxymethyltransferase fused to the amino acid sequence of activatable prochymosin. Novel polypeptides, which are expressed by transformants containing the expression vehicles, are also disclosed.

PRODUCTION OF BOVINE CALF CHYMOSIN

Chymosin (also known as rennin (EC 3.2.23.4) is a proteolytic enzyme that is abundant in the digestive tract of unweaned preruminant species. Chymosin is secreted as a zymogen, prochymosin, which is a single polypeptide chain containing 365 amino acids (40,700 daltons). Prochymosin is converted to chymosin by the specific removal of 42 N-terminal amino acids through a pH-dependent, autocatalytic reaction.

Bovine calf chymosin exists as two isozymes (designated A and B), which can be resolved by DEAE-cellulose chromatography of the crystalline enzyme. Isozyme B, which may be catalytically less efficient than isozyme A, is also the more abundant form in tissue extracts. Bovine calf chymosin isozyme B has been sequenced, and isozyme A has been partially sequenced, to reveal only one amino acid difference at residue 290 (glycine in B and aspartic acid in A) (see Foltmann, B. et al., J. Biol. Chem., 254:8447-8456 [1976]).

Bovine calf chymosin (mixture of isozymes) has been used for centuries in the manufacture of cheese. However, since World War II, a shortage of calf chymosin has developed, largely as a result of a decline in veal consumption. Accordingly, the chymosin obtained from abomasal tissue (rennet) of unweaned calves has steadily been replaced by other enzymes of animal and microbial origin none of which possess the optimal characteristics of the calf enzyme, particularly in cheese making.

As a result of this shortage, bovine calf chymosin has become a prime candidate for biosynthesis in transformed microorganisms produced by the techniques of DNA recombination. A cDNA sequence encoding bovine calf prochymosin, which was produced by reverse transcription from an mRNA preparation from the mucosal lining of the fourth stomachs of milk-fed calves, has been isolated and cloned.

The expression of DNA sequences encoding mammalian proteins in bacteria, under the control of regulatory sequences which normally direct the production of bacterial proteins, has been possible for several years. Unfortunately, some mammalian proteins have been difficult to express under the direct control of a bacterial protein regulatory sequence, either because the mammalian protein thus produced is quickly degraded within the bacterial environment or because the mammalian protein is only capable of very low levels of expression under the control of the bacterial regulatory sequence. Consequently, many mammalian proteins are expressed in the form of fusion proteins; that is, the DNA sequence encoding the amino acid sequence of the desired protein is joined, at the end corresponding to the N-terminus of the protein, to a DNA sequence encoding all or a portion of the bacterial protein which is normally under the control of the bacterial regulatory sequence. The fused bacterial/mammalian protein is efficiently expressed to produce a stable protein.

In the case of relatively small proteins such as insulin or polypeptide hormones, chemical cleavage of the fusion product to yield the desired protein is possible. With larger proteins, however, chemical cleavage of the fusion product is often accompanied by internal cleavage of the desired protein.

Recently, the production of chymosin by expression of a fusion protein in E. coli was attempted. A fusion protein was expressed in E. coli which contained almost all of the prochymosin sequence joined to a short N-terminal portion of E. coli β-galactosidase (Nishimori, K. et al., Gene, 19, 337 [1982]). The paper does not demonstrate activation of the fusion protein.

It is an object of the present invention to provide a method of expressing prochymosin at high levels of expression in a transformant microorganism, the expressed prochymosin having the ability to undergo autocatalytic conversion to chymosin.

It is another object of the invention to provide expression vectors and transformant microorganisms capable of high level production of prochymosin which is capable of undergoing autocatalytic conversion to chymosin.

It is yet another object of the invention to provide a method for efficiently producing chymosin using transformant microorganisms.

In accordance with the present invention, there is provided a replicable expression vehicle, preferably a plasmid, for expressing a polypeptide, which polypeptide is capable of undergoing autocatalytic cleavage to chymosin, comprising (a) a replicon; (b) a regulatory region corresponding to a regulatory region which directs expression, in a host microorganism, of a gene selected from the trpB gene and the glyA gene; and (c) a DNA sequence, operably linked to said regulatory region, comprising a sequence which encodes the amino acid sequence of an N-terminal fragment of a polypeptide selected from the β subunit of tryptophan synthetase (hereafter referred to as the "β subunit") and serine hydroxymethyltransferase, fused to the amino acid sequence of activatable prochymosin. When the regulatory region is one which controls the expression of the trpB gene, then the N-terminal fragment encoded by the DNA sequence which is operably linked thereto is an N-terminal fragment of the β subunit (i.e., the expression product of the trpB gene). Conversely, when the regulatory region is one which controls the expression of the glyA gene, then the N-terminal fragment which is operably linked thereto is an N-terminal fragment of serine hydroxymethyltransferase (i.e., the expression product of the glyA gene).

As used herein, the term "activatable prochymosin" refers to a polypeptide comprising the amino acid sequence of chymosin and at least a sufficient portion of the presequence of prochymosin such that the polypeptide is capable of undergoing autocatalytic coversion to chymosin.

We have found that the fusion protein which is expressed in a microorganism transformed with this expression vehicle is capable of undergoing autocatalytic cleavage to produce chymosin. The ability of the fusion protein to self-activate was somewhat surprising, since one might have expected the fusion to interfere with self-processing.

In one embodiment of the invention, a vector containing the trpB DNA sequence, encoding the amino acid sequence of the β subunit, operably linked to a regulatory sequence which directs expression of the trpB gene on a plasmid in the host microorganism, is cleaved at a first endonuclease restriction site within the sequence comprising the trpB gene and a synthetic oligodeoxynucleotide linker sequence is inserted into the cleavage site. The vector is then cleaved at a second endonuclease restriction site within the trpB gene sequence located downstream from the synthetic linker and a DNA sequence encoding the amino acid sequence of activatable prochymosin is inserted into the second cleavage site. The synthetic linker sequence is constructed so that it serves to keep the reading frame of the deoxynucleotides encoding prochymosin in phase with the reading frame established at the beginning of the trpB gene. The expression vehicle which is produced in this manner is used to transform a host microorganism. The microorganism is then grown up and subjected to conditions under which the encoded polypeptide is expressed.

The polypeptide which is expressed in this embodiment is a novel polypeptide comprising a first sequence of amino acid residues having the amino acid sequence of an N-terminal fragment of the $\beta$ subunit; a second sequence of amino acid residues having the amino acid sequence of activatable prochymosin; and a linker sequence which serves to fuse the first and second sequences at the carboxy terminus of the first sequence and the N-terminus of the second sequence. This polypeptide is capable of undergoing autocatalytic cleavage to produce chymosin.

The invention will now be described with reference to the accompanying drawings, in which;

Figure 1 represents a construction of plasmid pGx2213, which was modified to produce the replicable plasmidic expression vehicle of the invention.

Figure 2 represents the DNA sequence of the 5' end of the prochymosin coding region in plasmid pGx344, which was a source of DNA encoding the amino acid sequence of prochymosin employed to produce a replicable plasmidic expression vehicle of the invention.

Figure 3 represents the amino acid sequence of the N-terminal fragment of the $\beta$ subunit which was incorporated into a fusion protein of the invention, as well as its associated DNA sequence, which is represented in its proper reading frame. The last 17 bases include the restriction sites for insertion of the synthetic oligodeoxynucleotide linker sequence and the sequence encoding activatable prochymosin.

Figure 4 represents a modified segment of the trpB DNA sequence, after insertion of the synthetic oligodeoxynucleotide linker sequence, as well as the encoded amino acids.

Figure 5 represents the DNA sequence, as well as its associated amino acid sequence, at the fusion point of the trpB sequence and the prochymosin coding region in plasmid pGx2231.

Figure 6 represents a construction of the plasmid pGX2229 which was produced by insertion of the synthetic oligodeoxynucleotide linker sequence into pGX2223 (a close analog of pGx2213).

Figure 7 represents a construction of a replicable plasmidic expression vehicle of the invention, pGx2231.

The polypeptides of the invention can be produced by expression, in a transformed microorganism, of a replicable expression vehicle which is produced by the modification of a vector containing a trpB DNA sequence, which encodes the amino acid sequence of the β subunit, operably linked to a regulatory region which is capable of directing the expression of the trpB DNA. Alternatively, the polypeptides can be produced by expression, in a transformed microorganism, of a replicable expression vehicle which is produced by the modification of a vector containing a glyA DNA sequence, which encodes the amino acid sequence of serine hydroxymethyltransferase (SHMT), operably linked to a regulatory region which is capable of directing the expression of the glyA sequence. Suitable vectors include plasmids and phages, with plasmids being preferred. The criteria for selecting an expression vector include its size, location of expression control sequences with respect to the trpB or glyA sequence, and location and types of restriction endonuclease sites. With respect to size, the vector is preferably relatively small so as not to divert large amounts of cellular nutrients and energy to the production of unwanted macromolecules. Purification of the final product is also simplified, since a relatively small vector is unlikely to produce significant quantities of unwanted polypeptides which must be removed from the final product. Preferably, the vector is one which is capable of producing at least 10% of the total cell protein as the desired expression product. The vector must contain intact expression control sequences which remain functional after the insertion of the prochymosin DNA and which are located upstream of the trpB or glyA DNA sequence. It is preferred that the vector contain a unique restriction endonuclease site upstream from the expression control sequences.

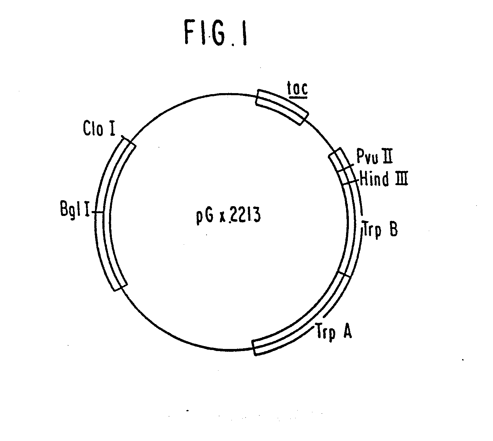

An expression vector which meets all of these requirements is the plasmid pGx2213 (Fig. 1). An E. coli strain (Gx1668) transformed by this plasmid has been deposited with the American Type Culture Collection in Rockville, Maryland as ATCC 39388. The plasmid is relatively small, 6456 base pairs. It carries the trpB DNA sequence, contains intact expression control signals (a trp/lac hybrid promoter, tac promoter) just upstream of the trpB DNA sequence, and contains a unique Bgl I restriction site located just upstream of the expression control signals. The plasmid also contains the trpA DNA sequence located immediately downstream from the trpB sequence, but contains none of the other genes of the tryptophan operon. Other expression vectors containing the trpB DNA sequence may also be employed in the practice of the invention.

The DNA sequence encoding the amino acid sequence of activatable prochymosin, which is inserted into the previously described vector, can be obtained from any convenient source. For example, it may be isolated directly from cleaved chromosomal DNA, in which case it is desirable to remove any introns from the DNA sequence to obtain a gene that is capable of functioning properly in a host microorganism such as E. coli. It may also be obtained by reverse transcription from mRNA enriched for the presence of prochymosin message.

The DNA sequence encoding the amino acid sequence of activatable prochymosin comprises a sequence encoding the amino acids of mature chymosin and at least a sufficient amount of the presequence such that the activatable prochymosin is capable of undergoing autocatalytic cleavage to produce chymosin. We have found that the nucleotide triplets encoding the two N-terminal amino acids of the presequence can be deleted with no deleterious effect, whereas deletion of the nucleotide triplets encoding the 28 N-terminal amino acids produces prochymosin which is only marginally capable of autocatalytic cleavage to produce active chymosin. We prefer to have no more than about 15 N-terminal nucleotide triplets deleted from the presequence, most preferably no more than six N-terminal triplets. If desired, the DNA sequence encoding the amino acid sequence of activatable prochymosin can include the entire sequence encoding prochymosin plus any portion of the sequence which encodes the signal peptide normally associated with the expression of prochymosin. The signal peptide, which precedes the 42 amino acid presequence, is a sequence of amino acids which is probably involved in transport of the protein through the cell membrane during secretion.

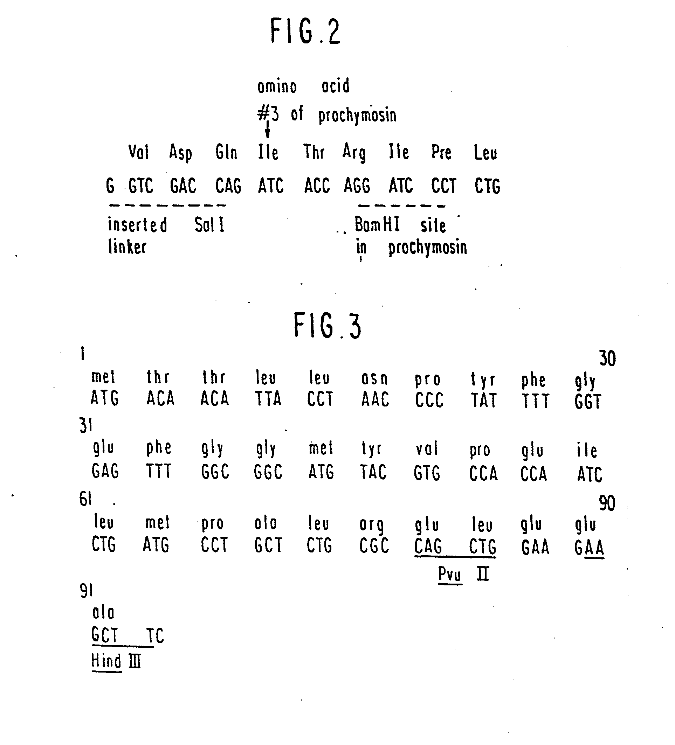

Preferably, the DNA sequence encoding activatable prochymosin is obtained from a cloning vector. The cloning vector is preferably a plasmid containing a unique restriction endonuclease site at the 5' end of the prochymosin sequence and a second unique restriction site located outside of and downstream from the prochymosin sequence. A suitable vector meeting these criteria is the plasmid pGx344. Fig. 2 depicts the 5' end of the sequence encoding prochymosin in pGx344. E. coli cells (strain HB101) transformed with this plasmid have been deposited with the American Type Culture Collection in Rockville, Maryland as ATCC 39389. This plasmid contains the DNA sequence encoding the amino acid sequence of bovine calf prochymosin (less the two N-terminal amino acids), a unique Sal I restriction endonuclease site located at the 5' end of the prochymosin sequence (see Fig. 2), and a unique Bgl I site outside of and downstream from the prochymosin sequence. In pGx344, codons for the sequence Val-Asp-Gln precede the codon for Ile, which is amino acid number 3 of native prochymosin. These additional codons are present as the result of the insertion of the SalI linker.

A replicable plasmidic expression vehicle of the invention can be produced by inserting the DNA sequence encoding the amino acid sequence of activatable prochymosin into an expression vector carrying the trpB DNA sequence. The insertion can be made at any point within the trpB coding region where a convenient site occurs, provided that a sufficient amount of the coding region corresponding to the N-terminal portion of the β subunit remains upstream of the inserted prochymosin coding region to allow efficient control of expression by the tryptophan regulatory region in the vector.

It is necessary that the DNA sequence encoding activatable prochymosin be inserted into the expression vector in such a manner that its reading frame is in phase with the regulatory region and the preceding oligodeoxynucleotides encoding the N-terminal fragment of the subunit. Accordingly, in one embodiment of the invention, prior to insertion of the DNA sequence encoding the amino acid sequence of prochymosin, the expression vector containing the trpB region is cleaved at an endonuclease cleavage site upstream of the insertion site of the prochymosin sequence and a synthetic oligodeoxynucleotide sequence is ligated to the ends of the cleavage site. The synthetic oligodeoxynucleotide has a sequence which serves to keep the reading frame of the prochymosin coding sequence in phase for the expression of the amino acid sequence of activatable prochymosin.

The trpB coding region into which the DNA sequence encoding activatable prochymosin is inserted, contains a Pvu II site located at base pairs 79-84, and a Hind III site located at base pairs 91-95 (see Fig. 3). We employed these sites, which are unique within the plasmid pGx2213, as the respective insertion sites for a synthetic oligodeoxynucleotide linker and the DNA sequence encoding the amino acid sequence of activatable prochymosin. The synthetic oligodeoxynucleotide linker is a double stranded DNA sequence which can be prepared using known procedures. The linker which we employed was a 10-base pair oligodeoxynucleotide in which the coding strand had the sequence:

5' CCA TCG ATG G 3'

When the DNA sequence having the synthetic oligodeoxynucleotide linker and the prochymosin coding region inserted therein is expressed, the resultant polypeptide, which is capable of undergoing autocatalytic cleavage to produce chymosin, comprises a first sequence of amino acid residues having the amino acid sequence of an N-terminal fragment of the β subunit; a second sequence of amino acid residues having the amino acid sequence of activatable prochymosin; and a linker sequence -- resulting from translation of the synthetic oligodeoxynucleotide region -- which serves to fuse the β subunit fragment and the prochymosin amino acid sequence. In a preferred embodiment of the invention, plasmid pGx2223 is linearized by digestion with Pvu II. Plasmid pGX2223 is essentially the same as pGX2213 that has been deposited at the American Type Culture Collection, Rockville, Maryland with accession number ATCC 39388. In plasmid pGX2223, the trpB and trpA DNA sequences downstream from the HindIII site have been replaced with another DNA sequence. The restriction enzyme cleaves the trpB coding region immediately after the guanine at position 81 of the DNA sequence (see Fig. 3). Consequently, the region encoding the first 27 amino acid residues at the N-terminus of the β subunit is preserved.

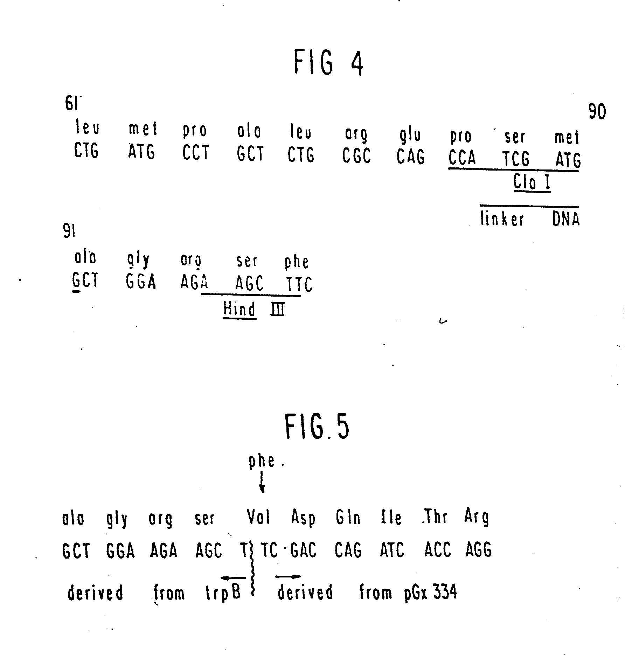

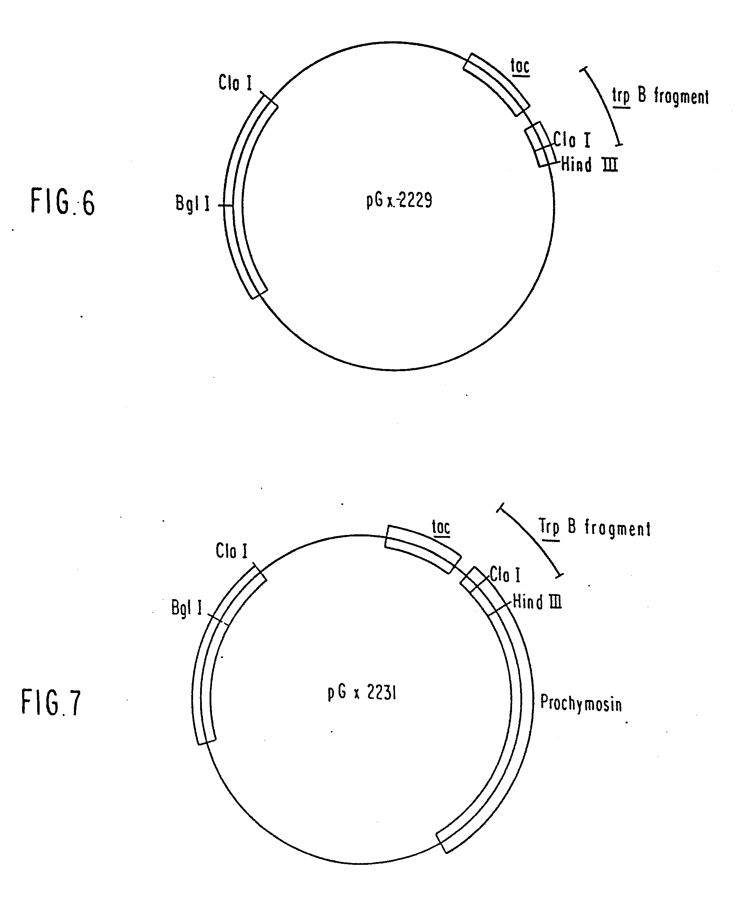

The previously mentioned 10-base pair synthetic oligodeoxynucleotide is then ligated to the ends of the linearized plasmid in the presence of ligating enzyme such as T4 ligase, resulting in a recircularized plasmid pGX2229 containing a linker sequence encoding a <u>Cla</u> I restriction site located within the <u>trpB</u> coding region just upstream from the <u>Hind</u> III site (see Fig. 4 and Fig. 6). Insertion of the linker sequence results in the following amino acid sequence which serves to fuse the N-terminal fragment of the β subunit to the activatable prochymosin:

PRO-SER-MET-ALA-GLY-ARG-SER-PHE

The recircularized plasmid pGX2229 is then digested with <u>Hind</u>III, leaving sticky ends. The sticky ends can be filled in by treatment with a mixture containing the enzyme DNA polymerase I (Klenow fragment), deoxyadenosinetriphosphate (dATP), deoxycytidinetriphosphate (dCTP), deoxyguanosinetriphosphate (dGTP), and deoxythymidinetriphosphate (dTTP). The resultant linearized plasmid has blunt ends. Although <u>Hind</u> III digestion cuts through the coding strand immediately after the Arg codon, removing the Ser and Phe codons, filling in restores the Ser codon and subsequent fusion with the cleaved SalI sequence in pGX344 at the 5' end of the prochymosin gene, as described below, restores the codon for Phe, which replaces the Val residue coded by the <u>Sal</u> I linker at the 5' end of the gene for activatable prochymosin in pGx344 (see Fig. 5).

When the plasmid pGx344 is used as the source of the activatable prochymosin DNA, it can be linearized by digestion with <u>Sal</u> I, leaving sticky ends. The sticky ends can be filled in by treatment in the same manner as described above with respect to pGx2229, resulting in a linearized plasmid with blunt ends.

A recombinant plasmid between the linearized pGx2229, containing the linker insert, and the linearized pGx344 can be most easily prepared by a two step ligation procedure. The plasmids are first blunt-end ligated at high DNA concentration (e.g., from about 0.2 mg/ml to about 2 mg/ml) to form heteropolymers of the two plasmids with some of the junctions of the desired type. Since pGx2229 and pGx344 are related plasmids each having a unique Bgl I site in the ampicillin resistance gene, the polymer is cut with Bgl I and then ligated at low DNA concentration (0.01 mg/ml to 0.001 mg/ml) to favor the reformation of plasmid circles.

When the linearized pGx2229 is ligated to the linearized pGx344, the resultant recombinant plasmid codes for a polypeptide consisting of the first 27 amino acids of the β subunit fused to the amino acid sequence of prochymosin (less the first two amino acids of its native sequence) through a linker sequence of amino acids. The linker sequence of amino acids consists of the aforementioned sequence which results from insertion of the ClaI linker followed by the two amino acids encoded by the residual codons from the SalI restriction site in pGx344, i.e., the amino acid sequence:

PRO-SER-MET-ALA-GLY-ARG-SER-PHE-ASP-GLN

A replicable expression vehicle of the invention which contains a regulatory region from the glyA gene and a sequence encoding the N-terminal fragment of SHMT (the glyA expression product) can be prepared in a manner analogous to that described above for the preparation of the expression vehicle incorporating the trpB regulatory region. As a suitable source of the DNA sequence comprising the glyA regulatory region and codons for the SHMT fragment, one can employ plasmid pGx139. A K. aerogenes strain (GX1704) transformed by this plasmid has been deposited with the American Type Culture Collection as ATCC 39214. This plasmid is similar to pGx2213, except that it is designed to overproduce SHMT, rather than tryptophan synthetase. A preferred source of DNA encoding the activatable prochymosin for use in this construction is plasmid pGx346. This plasmid is similar to previously described pGx344, except that it contains an additional 18 nucleotides, extending into the signal peptide coding region of preprochymosin, before insertion of the Sal I site.

Fortuitously, pGx139 contains an Xho I site within the region encoding SHMT which cleaves to produce a sticky end which is complementary to the end left by Sal I cleavage at the beginning of the activatable prochymosin gene in pGx346. Moreover, Xho I-cleaved pGx139 can be ligated to Sal I-cleaved pGx346 without any disruption in the reading frame. Accordingly, pGx139 and pGx346 can be cleaved and recombined to produce a replicable expression vehicle of the invention without the necessity of first inserting a linker sequence to adjust the reading frame.

Suitable host microorganisms are transformed with the recombinant plasmids using conventional methods, such as treatment in the presence of $CaCl_2$ at 4°C to render the cell walls permeable to the recombinant plasmid. Transformants containing the desired DNA in the proper orientation for expression of the polypeptides of the invention can be identified by assaying the expression product or by immunological detection methods. The desired recombinants can also be readily detected by examining the colony morphology of the transformants growing on petri dishes. Chymosin producing transformants containing plasmids with control elements that result in high level constitutive expression (such as the control elements of plasmids pGx2213 and pGx139) produce inclusion bodies within the cells. The inclusion bodies give the colonies a more dense white appearance that can be readily distinguished from colonies that are not producing inclusion bodies.

The identified transformants containing the desired DNA sequences can be grown up under fermentation conditions for production of the fusion polypeptide. The exact fermentation conditions utilized are dependent upon the particular regulatory region utilized in the recombinant plasmid. Plasmid pGx2231 (constructed from pGx2213 and pGx344) and pGx2234 (constructed from pGx139 and pGx346)

will produce the polypeptide of the invention constitutively.

Preferred host microorganisms for use in the invention include species of the <u>genera</u> <u>Escherichia</u>, <u>Klebsiella</u> and <u>Salmonella</u>. Particularly preferred microorganisms are <u>E. coli</u> and <u>K. aerogenes</u>. The polypeptide can be recovered from the transformant microorganism, either under conditions which induce the polypeptide of the invention to autocatalytically cleave to yield chymosin directly, or under conditions which do not result in the cleavage of the polypeptide. As it is initially produced, the fusion product is found in the form of highly insoluble inclusion bodies within the cell. This material can be purified from the majority of the cellular proteins by mechanically disrupting the cells and collecting the insoluble material by centrifugation. If it is desired to recover the product in the form of active mature chymosin, the expressed polypeptide (i.e. the fusion of the N-terminal fragment of the $\beta$ subunit and the activatable prochymosin) is extracted into a denaturing solvent such as NaOH, urea or guanidine. The polypeptide is then renatured by diluting or removing the denaturing agent in the solvent. When NaOH is used as the denaturing agent, the polypeptide is preferably extracted into an NaOH solution at a pH of at least 12.0 and renaturation is effected by diluting the solution and incubating the diluted solution at pH 8.5 to 10.5. When urea or guanidine is used as the denaturing agent, renaturation is preferably effected by dialyzing the solution to remove the urea or guanidine. Following renaturation the pH of the solution containing the renatured polypeptide is reduced, preferably to below 2.0, which results in conversion of the polypeptide to enzymatically active pseudochymosin. The active pseudochymosin may be employed directly as an enzyme or it may be converted to active mature chymosin by elevating the pH of the pseudochymosin solution to between 4.0 and 7.0 preferably about 6.0.

The chymosin, or the fusion product, can be purified using known techniques of protein purification, e.g., chromatographic techniques such as reverse phase liquid chromatography, size exclusion chromatography or immunoaffinity chromatography.

The present invention is further illustrated by the following examples, which are not intended to be limiting.

## Example I

Plasmid pGx2223, a close analog of plasmid pGX2213 (Fig. 1) was linearized by digestion with Pvu II. Pvu II and other enzymes used in the examples were obtained commercially and used according to the manufacturer's specifications. The Pvu II-cut pGx2223 DNA solution was extracted with a mixture of phenol-chloroform (1:1) saturated with water at pH 8.0. The aqueous layer was made 0.2M in sodium acetate pH 5.5 and 2.5 volumes of ethanol were added. The solution was frozen on dry ice then centrifuged at 12,000 x G to precipitate the DNA. The pellet was dried in vacuo and dissolved in water. The extraction and precipitation procedure above was utilized after each enzyme step in all the methods described below. The Pvu II-cut pGx2223 DNA was treated with bacterial alkaline phosphatase. The alkaline phosphatase treated DNA (4 μg) was mixed and ligated with 130μ g of phosphorylated Cla I linker DNA (5'-CCATCGATGG-3') in 10 μl for 12 hours at 12°C with 1 unit of T4 polynucleotide ligase and 0.5 mM ATP. The ligation mixture was passed over a Bio Gel A50M gel filtration column to remove excess linkers. The

pGx2223 DNA was collected in the void volume of the column, ethanol precipitated as described above and digested with Cla I endonuclease. Some of the DNA (1 μg) was then ligated to recircularize in a volume of 200 ml buffer for 12 hours with 1 unit of T4 ligase and 1 mM ATP at 12°C. The ligation mixture was used to transform E. coli by conventional techniques and spread onto LB media (10g/liter tryptone, 5g/liter yeast extract, 10g/liter NaCl, and 1.5% agar), with 100 μg/ml ampicillin. Plasmids were screened for the presence of a unique Cla I site. One plasmid was chosen and designated pGx2229.

Plasmid pGx2229 was linearized with Hind III and treated with E. coli DNA polymerase I (Klenow fragment) in the presence of dATP, dCTP, dGTP and dTTP each at a concentration of 0.5 mM for 1 hour at 12°C. The result was a linearized plasmid with blunt ends.

Plasmid pGx344, containing the DNA sequence for prochymosin less the two N-terminal amino acids, was digested with Sal I endonuclease resulting in a linearized plasmid with sticky ends. After extraction and ethanol precipitation the DNA was treated with DNA polymerase to make blunt ends by the same procedure described for Hind III-cut pGx2229 above.

The linearized plasmids pGx2229 and pGx344 with blunt ends were ligated at high concentration by adding 2 μg of each to a 10 μl ligation reaction with 1 unit of T4 ligase and 0.5 mM ATP and incubating at 12°C for 12 hours. After extraction and precipitation the ligation mixture was digested with Bgl I, then 1 μg of the Bgl I-cut DNA was diluted into a 200 μl ligation reaction with 1 unit of T4 ligase, 1 mM ATP at 12°C for 12 hours.

E. coli strain Gx1670 (contains a lacIq1 mutation) was transformed with the ligation mix and plated on LB with 100 μg/ml ampicillin. Candidate transformants for containing the desired fusion plasmids were grown on LB-ampicillin plates in a grid pattern. After overnight growth a nitrocellulose filter was touched to the surface of the plate to
lift colonies. The filter was sprayed with 10 mg/ml lyso-
zyme in 0.25 M Tris pH 8.0 and incubated at 20°C for 10
minutes. The filter was then sprayed with chloroform and
dried. The filters were pretreated with TS (10 mM Tris pH
7.5, 0.9% NaCl) with 5% BSA (Bovine serum albumin) then
treated with TS with BSA plus rabbit anti-chymosin antibody. The filter was washed completely with TS, then
treated with goat anti-rabbit IgG antibody that was coupled
with peroxidase. After washing again with TS, the chymosin
antigen containing colonies were identified by adding 0.5
mg/ml 4-chloro-1-naphthol in 20% methanol with 0.06% $H_2O_2$.

Alternatively, prochymosin fusion producing colonies
can be identified visually by careful examination of plates
with indirect light from a diffused light source. Prochymosin fusion producing colonies have a much more dense
white appearance than non-producing colonies.

One colony identified by both immunoassay and appearance contained plasmid pGx2231 (Fig. 7).

Colonies containing pGx2231 were grown in LB ampicil-
lin (100 $\mu$ g/ml) liquid medium in shaker flasks (500 ml per
3 liter flask) at 37°C to stationary phase. Cells were
harvested by centrifugation at 500 x G and washed with
water. The cells were lysed by sonification and centrifuged. The pellet fraction was resuspended in water and
centrifuged again. Essentially 100% of the prochymosin
fusion protein is found in the pellet fraction and is substantially purified to at least 40% pure. Activation of
the purified fusion protein into functional chymosin can be
effected by the method described above.

Example II

Plasmid pGx139 (ATCC 39214) is similar to pGx2213
described in Fig. 1, except it is designed to overproduce serine hydroxymethyltransferase (SHMT) rather than tryptophan synthetase. The glyA gene, which codes for SHMT, and its regulatory region are inserted in pGx139 in the same relative orientation as the trpBA gene in pGx2213. The nucleotide sequence and restriction map of the glyA gene are described in Plamann, et al., Nucleic Acids Research, 11, 2065-2075. Plasmid pGx346 is a close analog of pGx344. It is identical except for the fact that it contains 18 more nucleotides of the natural chymosin gene before the insertion of the Sal I linker. Thus, it has the complete code for prochymosin plus four amino acids of the prepro-chymosin region.

Plasmid pGx139 was digested with Xho I, which makes a unique cut in the glyA gene. Plasmid pGx346 was digested with Sal I, which makes a unique cut at the beginning of the chymosin gene. Xho I and Sal I produce the same sticky end, and in this case joining the glyA and chymosin fragments produces a fused gene with a consistent reading frame that codes for a protein containing 301 amino acids of SHMT fused to the preprochymosin amino acids of pGx346. The Xho I-cut pGx139 and Sal I-cut pGx346 (2 $\mu$g of each) were ligated in 10 $\mu l$ at 12°C with 1 mM ATP and 1 unit of T4 ligase for 12 hours. The ligation mixture was phenol-chloroform extracted and ethanol precipitated as described in Example I. In order to form plasmid circles, the DNA was digested with Bgl I and ligated at low concentration (approximately 1 $\mu$g/200 $\mu$l reaction volume) as described in Example I. The ligation mixture was used to transform E. coli, strain Gx1670. No special genotype of E. coli is required for this construction and prochymosin fusion producing colonies were identified as described in Example I. The plasmid in one fusion producing transformant was identified as pGx2233.

Cells containing plasmid pGx2233 were grown in a fashion identical to those described for plasmid pGx2231 in Example I. The chymosin fusion product again was found to be an insoluble material that could be greatly purified by centrifugation after cell lysis. Further, this material could be activated to functional chymosin using the same methods as for the expression product of pGx2231.

## Example III

The two step ligation procedure described in Examples I and II was applied to the construction of another trpB prochymosin fusion using pGx2229 described in Example I and pGx346 described in Example II. Some of the same pGx2229 DNA prepared by Hind III digestion and DNA polymerase treatment described in Example I was ligated at high concentration to some pGx346 that had been made blunt ended with DNA polymerase after Sal I digestion (2 $\mu$g of each DNA in a 10 $\mu$l reaction volume as described in Example I). The DNA was extracted, precipitated and digested with Bgl I before ligation at low concentration. The ligation mixture was used to transform E. coli Gx1670, and chymosin fusion producing colonies were identified as described in Example I. The plasmid from one positive colony was identified as pGx2232. Cells containing pGx2232 were grown in an identical fashion to cells with pGx2231. The prochymosin fusion material was again found to be in an insoluble state that facilitates purification and is capable of being activated to functional chymosin.

When an identical construction to those described in Examples I and II was prepared with plasmid pGx323 as the prochymosin donor, insoluble fusion material was produced, but it could only be marginally activated into functional prochymosin. The chymosin DNA in pGx323 is 76 bases shorter than pGx344 and 94 bases shorter than the DNA in pGx346. Although this structure still has the code for 15 amino acids of the natural prochymosin presequence, only a marginal amount of functional chymosin is obtained by self processing with the product of this construction.

CLAIMS:

1. A polypeptide which is capable of undergoing autocatalytic cleavage to produce chymosin which comprises:

(a) a first sequence of amino acid residues having the amino acid sequence of an N-terminal fragment of the β subunit of tryptophan synthetase or of a N-terminal fragment of serine hydroxymethyltransferase.

(b) a second sequence of amino acid residues having the amino acid sequence of activatable prochymosin; and

(c) a linker sequence which serves to fuse said first and second sequences at the carboxy terminus of said first sequence and the N-terminus of said second sequence.

2. A polypeptide as claimed in claim 1, wherein the N-terminal fragment of the β subunit of tryptophan synthetase in the polypeptide has the amino acid sequence

Met-Thr-Thr-Leu-Leu-Asn-Pro-Tyr-Phe-Gly-

Glu-Phe-Gly-Gly-Met-Tyr-Val-Pro-Glu-Ile-

Leu-Met-Pro-Ala-Leu-Arg-Glu

3. A polypeptide as claimed in claim 2, wherein the linker sequence which fuses the β subunit fragment to the amino acid sequence of prochymosin has the sequence Pro-Ser-Met-Ala-Gly-Arg-Ser-Phe-Asp-Gln

4. A replicable plasmidic expression vehicle for expressing a polypeptide, which polypeptide is capable of undergoing autocatalytic cleavage to produce chymosin, comprising:

(a) a replicon;

(b) a regulatory region which is capable of directing expression of a gene selected from trpB and glyA in a host micro organism; and

(c)    a DNA sequence, operably linked to said regulatory region, comprising a sequence which encodes the amino acid sequence of an N-terminal fragment of:

(i)    the    subunit of tryptophan synthetase, when the regulatory region is one which is capable of directing expression of <u>trpB</u>;   or

(ii)    serine hydroxymethyltransferase, when the regulatory region is one which is capable of directing expression of <u>glyA</u>,

said N-terminal fragment being fused to the amino acid sequence of activatable prochymosin.

5.    A replicable plasmidic expression vehicle as claimed in claim 4, wherein the regulatory region is one which directs expression of the <u>trpB</u> gene and the DNA sequence operably linked thereto encodes the N-terminal fragment of the    subunit of tryptophan synthetase.

6.    A replicable plasmidic expression vehicle as claimed in claim 5, wherein the sequence encoding the N-terminal fragment of the    subunit of tryptophan synthetase is linked to the sequence encoding activatable prochymosin through a linker sequence which serves to keep the reading frame of the prochymosin-encoding sequence in phase with the regulatory region and sequence encoding the N-terminal fragment of the    subunit of tryptophan synthetase.

7.    A replicable plasmidic expression vehicle as claimed in claim 6 which is pGx2231.

8.    A replicable plasmidic expression vehicle as claimed in claim 4, wherein the regulatory region is one which directs expression of the <u>glyA</u> gene and the DNA sequence operably linked thereto encodes an N-terminal fragment of serine hydroxymethyltransferase.

9.    A transformant micoorganism for the expression of a polypeptide, which polypeptide is capable of undergoing autocatalytic cleavage to produce chymosin, said microorganism being transformed by the replicable plasmidic expression vehicle of claim 4.

10. A transformant microorganism for the expression of a polypeptide, which polypeptide is capable of undergoing autocatalytic cleavage to produce chymosin, said microorganism being transformed by the replicable plasmidic expression vehicle of claim 5.

11. A transformant microorganism for the expression of a polypeptide, which polypeptide is capable of undergoing autocatalytic cleavage to produce chymosiin, said microorganism being transformed by the replicable plasmidic expression vehicle of claim 6.

12. A transformant as claimed in claim 9, wherein the replicable plasmidic expression vehicle is the plasmid pGx2231.

13. A transformant microorganism for the expression of a polypeptide, which polypeptide is capable of undergoing autocatalytic cleavage to produce chymosin, said microorganism being transformed by the replicable plasmidic expression vehicle of claim 8.

14. A method of producing a polypeptide which is capable of undergoing autocatalytic cleavage to produce chymosin which comprises:

(a) growing up a transformant microorganism as claimed in claim 9;

(b) subjecting the microorganism to conditions which initiate expression of the polypeptide which is under the control of the regulatory region which directs expression of the trpB or glyA fragment; and

(c) recovering the polypeptide.

15. A method as claimed in claim 14, wherein the transformant is transformed with a replicable plasmidic expression vehicle as claimed in claim 5.

16.   A method as claimed in claim 14, wherein the transformant microorganism has been transformed by the plasmid pGx2231.

18.   A method of producing a replicable expression vehicle for the production of a polypeptide, which polypeptide is capable of undergoing autocatalytic cleavage to produce chymosin, comprising:

(a)   providing a replicable vector containing a regulatory sequence which directs expression in a host microorganism, of a DNA sequence selected from trpB and glyA, said regulatory sequence being operably linked to a DNA sequence encoding the amino acid sequence of:

(i)   the β subunit of tryptophan synthetase, when the regulatory region is one which is capable of directing expression of trpB;   or

(ii)   serine hydroxymethyltransferase, when the regulatory region is one which is capable of directing expression of glyA;

(b)   cleaving said vector at an endonuclease cleavage site located within the sequence encoding the amino acid sequence of the β subunit or serine hydroxymethyltransferase; and

(c)   inserting into the cleavage site a DNA sequence containing a sequence of deoxynucleotides encoding the amino acid sequence of bovine calf prochymosin, the reading frame of the deoxynucleotides encoding the bovine calf prochymosin being in-phase with respect to the regulatory sequence and the preceding portion of the DNA sequence encoding the β subunit or serine hydroxymethyltransferase to direct expression of a polypeptide comprising prochymosin fused to an N-terminal fragment of the β subunit or serine hydroxymethyltransferase.

19.    A method as claimed in claim 18 wherein said vector is a plasmid.

20.    A method as claimed in claim 18, wherein, prior insertion of said DNA sequence encoding the amino acid sequence of prochymosin, the plasmid vector is cleaved at endonuclease cleavage site within the trpB sequence upstream of the insertion site of the prochymosin coding sequence and a synthetic deoxynucleotide is ligated to the ends of the cleavage site, said synthetic deoxyoligonucleotide having a sequence which serves to keep the reading frame of the prochymosin coding sequence in phase for the expression of the amino acid sequence of prochymosin.

21.    A method as claimed in claim 20, wherein said synthetic deoxyoligonucleotide is ligated to the Pvu II cleavage site within the trpB sequence and the prochymosin coding sequence is inserted at the Hind III site within the trpB sequence.

22.    A method as claimed in claim 21, wherein the coding strand of the synthetic deoxyoligonucleotide has the sequence

5' CCA TCG ATG G 3'

23.    A polypeptide, capable of undergoing autocatalytic cleavage to produce chymosin, which comprises:

(a)    a first sequence of amino acid residues having the amino acid sequence of an N-terminal fragment of serine hydroxymethyltransferase; and

(b)    a second sequence of amino acid residues having the amino acid sequence of activatable prochymosin; said sequences being joined at the carboxy terminus of the first sequence and the N-terminus of the second sequence.

FIG.1

## FIG.2

amino acid
#3 of prochymosin
↓

| Vol | Asp | Gln | Ile | Thr | Arg | Ile | Pre | Leu |
|-----|-----|-----|-----|-----|-----|-----|-----|-----|
| G GTC | GAC | CAG | ATC | ACC | AGG | ATC | CCT | CTG |

inserted    Sal I                    .. BamHI  site
linker                               in  prochymosin

## FIG.3

1                                                                                          30
met   thr   thr   leu   leu   asn   pro   tyr   phe   gly
ATG   ACA   ACA   TTA   CCT   AAC   CCC   TAT   TTT   GGT

31
glu   phe   gly   gly   met   tyr   vol   pro   glu   ile
GAG   TTT   GGC   GGC   ATG   TAC   GTG   CCA   CCA   ATC

61  .                                                                                      90
leu   met   pro   ola   leu   arg   glu   leu   glu   glu
CTG   ATG   CCT   GCT   CTG   CGC   CAG   CTG   GAA   GAA
                                          Pvu  II

91
ola
GCT    TC
Hind III

# FIG 4

61                                                  90

| leu | met | pro | ala | leu | arg | glu | pro | ser | met |
|-----|-----|-----|-----|-----|-----|-----|-----|-----|-----|
| CTG | ATG | CCT | GCT | CTG | CGC | CAG | CCA | TCG | ATG |

Clo I

linker DNA

91

| ala | gly | arg | ser | phe |
|-----|-----|-----|-----|-----|
| GCT | GGA | AGA | AGC | TTC |

Hind III

# FIG.5

phe.
↓

| ala | gly | arg | ser | Val | Asp | Gln | Ile | Thr | Arg |
|-----|-----|-----|-----|-----|-----|-----|-----|-----|-----|
| GCT | GGA | AGA | AGC | T⟩TC | GAC | CAG | ATC | ACC | AGG |

derived from trpB ⟩ derived from pGx 334

FIG.6

FIG.7

## DOCUMENTS CONSIDERED TO BE RELEVANT

EP 84304688.9

| Category | Citation of document with indication, where appropriate, of relevant passages | Relevant to claim | CLASSIFICATION OF THE APPLICATION (Int. Cl.4) |
|---|---|---|---|
| A,D | GENE, vol. 19, 1982 (Amsterdam, NL)<br><br>K. NISHIMORI et al. "Expression of cloned calf prochymosin gene sequence in Escherichia coli" pages 337-344<br><br>* Summary *<br>-- | 1,4 | C 12 P 21/02<br>C 12 N 15/00<br>C 12 N  9/48<br>C 07 K  7/10 |
| A | EP - A2 - 0 068 691 (CELLTECH LIMITED)<br><br>* Abstract *<br>-- | 1,4 | |
| A | EP - A2 - 0 057 350 (COLLABORA-TIVE RESEARCH INC.)<br><br>* Abstract *<br>-- | 1,4 | |
| A | EP - A2 - 0 077 109 (UNILEVER NV)<br><br>* Abstract; claims 1,9 *<br>---- | 1,4 | **TECHNICAL FIELDS SEARCHED (Int. Cl.4)**<br><br>C 12 P<br>C 12 N<br>C 07 K |

The present search report has been drawn up for all claims

| Place of search | Date of completion of the search | Examiner |
|---|---|---|
| VIENNA | 09-10-1984 | WOLF |